# EUROPEAN PATENT APPLICATION

(11) **EP 2 510 872 A1**
(43) Date of publication of application: **17.10.2012**
(21) Application number: 12159234.9
(22) Date of filing: 13.03.2012
(51) Int. Cl.: A61B 5/0464, A61M 5/172, A61N 1/362, A61N 1/39, A61M 5/142

(54) **Method of determination of a type of arrhythmia in a heart of a patient and respective device**

(30) Priority: 13.04.2011 US 201161474755 P
(71) Applicant: BIOTRONIK SE & Co. KG, 12359 Berlin (DE)
(72) Inventor: Lian, Jie, Beaverton, Oregon 97007 (US); Lang, Volker, West Linn, Oregon 97068 (US)
(74) Representative: Lindner-Vogt, Karin L.

(57) **Abstract**

The invention refers to a method of fast, reliable as well as high sensitive and specific determination of a type of an atrial and ventricular arrhythmia in a patient's heart, the method comprising:
● monitoring the atrial and ventricular rate of the heart;
● detecting a pathological initial ventricular and/or atrial rate during a first time period;
● administering, preferably intravenous administering, of at least one antiarrhythmic cardioactive drug only over a second time period, preferably over a short time period of 1 to 10 seconds, in case that a pathological initial ventricular and/or atrial rate is detected;
● detecting the response of the ventricular and atrial rate of the heart to the administerring of the at least one drug, comparing the ventricular and atrial rate response with the initial ventricular and atrial rate, respectively, within a third time period and determining whether there are changes within the ventricular and/or atrial rate response and determining the type of changes; and
● determining the type of atrial or ventricular arrhythmia from the presence or absence of changes of the atrial and/or ventricular rate response compared with the initial atrial and ventricular rate and from the type of these changes.

The invention further refers to an appropriate device comprising an implantable cardiac device (10) and a drug delivery device (20).

## Description

The invention relates to a method of determination of a type of arrhythmia in a patient's heart and a respective device comprising an implantable cardiac device, for example a pacemaker, defibrillator or cardioverter.

An arrhythmia is an abnormal heart rhythm. One example of an arrhythmia includes a tachyarrythmia wherein the heart beats at an abnormally fast rate. With atrial tachycardia the atria of the heart beat abnormally fast and with ventricular tachycardia the ventricles of the heart beat abnormally fast. Though often unpleasant for the patient an atrial tachycardia is typically not fatal. However, some tachycardia, particularly ventricular tachycardia, can trigger ventricular fibrillation wherein the heart beats chaotically resulting in little or no net flow of blood from the heart to the brain and organs. Ventricular fibrillation, if not terminated, may be fatal. Hence, it is highly desirable to prevent or terminate arrhythmia, particularly ventricular tachycardia, with an appropriate therapy.

Implanted medical devices that exist today are capable of detecting and treating arrhythmia of a patient. For that, an implanted medical device includes for example a defibrillator which applies an electrical therapy to a patient's heart upon detecting an atrial fibrillation. Cardioverters or defibrillators discharge relatively high energetic electrical shocks across cardiac tissue to arrest a life threatening ventricular fibrillation that is detected by the implanted medical device. Defibrillation shocks while highly effective at arresting the fibrillation may cause considerable patient discomfort and should therefore only be applied if they are really necessary. Other therapy of tachycardia or fibrillation includes drug administration, wherein the drugs are often highly specific with regard to the type of tachycardia or fibrillation.

In order to apply the most appropriate therapy to the patient suffering an arrhythmia it is necessary to know the type of an arrhythmia occurring with a patient very precisely. For example, if high ventricular rate is accompanied by high atrial rate, than a decision needs to be made to distinguish SVT (Supra Ventricular Tachycardia) from VT (Ventricular Tachycardia). Although other criteria, such as sudden onset, stability, AV consistency, QRS morphology, etc. of a cardiac signal, can be used to facilitate the classification, the sensitivity and specificity of those methods are not sufficiently high. Further, those methods need quite a long time to make the decision.

Hence, the object of the invention is to provide an improved method and an improved device with which a reliable, fast as well as highly sensitive and specific determination of the type of arrhythmia can be made in order to apply the most appropriate therapy to the patient shortly after detection of the arrhythmia.

Document US 2004/0267321 A1 describes an implantable cardiac stimulation device which is configured to automatically monitor the effects of antiarrhythmic drugs on cardiac electrical signals within a patient to verify efficacy of the drugs taken. The implantable cardiac stimulation device disclosed in US 2004/0267321 utilizes atrial and ventricular sensing circuits to sense cardiac electric signals to determine whether a rhythm is physiologic or pathologic. The sensed signals are than processed in order to determine the presence of an arrhythmia. Therein, timing intervals between sensed events (e.g., P-waves, R-waves, and depolarization signals associated with fibrillation which are sometimes referred to as "F-waves" or "FIB-waves") are classified by a microcontroller by comparing them to a predefined rate zone limit and various other characteristics (e.g., sudden onset, stability, physiologic sensors, and morphology, etc.). The known device further includes an antiarrhythmic drug efficacy monitoring unit for automatically monitoring the efficacy of antiarrhythmic drugs prescribed to the patient. Therefore, the monitoring unit includes a cardiac signal analysis unit for analyzing the patient's cardiac signal to verify the efficacy of the prescribed drugs and a warning signal generation unit for generating a warning signal alerting the patient or physician to possible drug efficacy problems. The monitoring unit also includes a drug pump control unit for automatically controlling an optional implantable drug pump to compensate, if necessary, for drug efficacy problems. For example, if an initial dosage of an antiarrhythmic drug is not adequately effective, the drug pump maybe controlled to increase the dosage. Additionally, the known device comprises a monitoring unit including a control parameter adjustment unit for automatically adjusting pacing control parameters used by the implanted device to compensate for drug efficacy problems. For example, if the prescribed antiarrhythmic drug is not adequately effective overdrive pacing control parameters can be adjusted to increase the aggressiveness of overdrive pacing.

From above summary of US 2004/0267321 A1 it can be derived that the known device refers to a slow state of the art arrhythmia determination and a long-term stabilization of antiarrhythmic drug delivery. The state of the art device does not solve above mentioned problem.

US 6,968,226 B2 is directed to a method and system for automatic intervention by an implanted device to prevent and/or terminate an atrial arrhythmia in a patient's heart using pacing and/or pharmaceutical therapies. The known device measures at least on electriccardiogram characteristic indicative of an atrial arrhythmia, thereby detecting an atrial arrhythmia of the heart, and thereafter transmitting a warning signal to the patient. Instead of, or in addition to, the traditional electrical cardioversion therapy, the patient chooses a pharmaceutical therapy via an available drug delivery arrangement, for example via an external drug delivery arrangement (e.g. intravenously, orally, transdermally, intramuscularly, orally, inhalationally, among others) to terminate the atrial fibrillation. While delivering the drug therapy or shortly thereafter the implanted medical device measures at least one additional Q-T interval and denominates it as the drug therapy Q-T interval. Drug therapy is terminated if a drug therapy Q-T interval is measured to be greater than a drug therapy Q-T interval threshold. For example, drug therapy is stopped if the Q-T interval after initiation of drug therapy is measured to exceed 500 ms. This document describes a slow determination of arrhythmic type and a long term therapy of arrhythmia as well and does not address the above mentioned problem either.

The above object is achieved by a method described in claim 1 and a device according to claim 8.

The above object is achieved in particular by a determination method of a type of an atrial or ventricular arrhythmia in a patient's heart, the method comprising:
- monitoring the atrial and ventricular rate of the heart;
- detecting a pathological initial ventricular and/or atrial rate during a first time period;
- administering, preferably intravenous administering, of at least one antiarrhythmic cardioactive drug over a second time period, preferably over a short time period of 1 to 10 seconds, in case that a pathological initial ventricular and/or atrial rate is detected;
- detecting the response of the ventricular and atrial rate of the heart to the administering of the at least one drug, comparing the ventricular and atrial rate response with the initial ventricular and atrial rate, respectively, within a third time period and determining whether there are changes within the ventricular and/or atrial rate response and determining the type of changes;
- determining the type of atrial or ventricular arrhythmia from the presence or absence of changes of the atrial and/or ventricular rate response compared with the initial atrial and ventricular rate and from the type of these changes.

The above object is further achieved in particular by a device for determination of a type of an atrial and ventricular arrhythmia in a patient's heart comprising an implantable cardiac device and a drug delivery device, wherein:
- the cardiac device is adapted to monitor the atrial and ventricular rate of the heart;
- the cardiac device is adapted to detect a pathological initial ventricular and/or atrial rate during a first time period;
- the drug delivery device is adapted to administer, preferably intravenous, of at least one antiarrhythmic cardioactive drug only over a second time period, preferably over a short time period of 1 to 10 seconds, in case that a pathological initial ventricular and/or atrial rate is detected;
- the cardiac device is adapted to detect the response of the ventricular and atrial rate of the heart to the administering of the at least one drug, to compare the ventricular and atrial rate response with the initial ventricular and atrial rate, respectively, within a third time period and to determine whether there are changes within the ventricular and/or atrial rate response and to determine the type of changes; and
- the cardiac device is adapted to determine the type of atrial or ventricular arrhythmia from the presence or absence of changes of the atrial and/or ventricular rate response compared with the initial atrial and ventricular rate and from the type of these changes.

The above proposed inventive method and device use the property of certain drugs influencing the ventricular or atrial rate highly effectively and specific with regard to a certain type of arrhythmia. Hence, it is possible to determine the type of atrial or ventricular arrhythmia fast and with high sensitivity and specificity. Correspondingly, appropriate therapy can be initiated with improved efficacy.

The present invention is based on the idea, that a high efficient drug is administered over a very short time only in order to observe the respective response of the heart so that the type of arrhythmia can be determined. Using the proposed short diagnostic drug pulse, which comprises a considerable smaller amount than a therapeutic dosage, on the one hand the determination of the type of arrhythmia can be made fast and on the other hand a probably negative effect to the heart behavior or health of the patient can be avoided. Further, only after a correct and reliable determination of the type of arrhythmia the most effective therapy for the patient can be initiated.

In this application an antiarrhythmic cardioactive medicinal product (one or more medicament, drug, steroid, monoclonal antibody, not capsuled or at least one of them capsuled, if necessary containing additives and/or matrix substances), in the following referred to as antiarrhythmic cardioactive drug (or short: drug), is administered preferably intravenously or directly into the heart.

The cardiac device in one embodiment comprises a control unit which processes data, a detection unit for monitoring of the atrial and ventricular rate, a memory unit, and a power supply. The control unit controls the drug delivery device in order to administer the at least one antiarrhythmic cardioactive drug and processes data received from the detection unit, in particular measured atrial and ventricular rate of the patient's heart. Therefore the drug delivery device comprises a pump and at least one drug reservoir. In another embodiment the cardiac device further comprises electrodes for application of pacing or shock energy to the heart and/or the drug delivery device comprises further drug reservoirs in order to apply therapeutical drug doses.

In an embodiment, the second time period of administering of the at least antiarrhythmic cardioactive drug takes 1 to 3 seconds and the drug delivering device is adapted to administer this drug during the proposed time interval. This time interval is usually sufficient for the proposed diagnostic utilization of the drug in order to get the appropriate response of the ventricular and atrial rate of the heart to determine the type of atrial and ventricular arrhythmia.

In another embodiment the at least antiarrhythmic cardiactive drug comprises adenosine. Adenosine is a naturally occurring nucleoside that, when given intravenously, has a rapid peak effect (10 to 30 seconds) manifested by transient high degree AV block and profound slowing of the AV node, or both. Moreover, it is removed from the circulation very quickly, and its half-life is less than 10 seconds. Furthermore adenosine is usually well tolerated by the patients. These characteristics make adenosine highly effective for the determination of the type of atrial tachycardia, in particular for acute determination of the AV nodal dependent tachyarrhythmias. Furthermore, not only an AV nodal dependent tacharrhythmia can be determined but as a by-product of the diagnostic drug release, often AV nodal reentry and bypass-tract-mediated reentry arrhythmia can be terminated by an intravenous bolus of adenosine. Although, adenosine may not terminate other types of SVT that do not depend on the AV node, such as atrial tachycardia, atrial fibrillation, and atrial flutter, the transient AV block caused by adenosine can transiently slow atrial tachyarrhythmias. For a ventricular tachycardia, however, adenosine, generally has no effect. Accordingly, in one embodiment an adenosine bolus is utilized to discriminate between AV nodal dependent SVT, SVT (not AV nodal dependent) and VT arrhythmia as described below.

As a result, many inappropriate therapies (antitachycardia pacing or shock) can be avoided. While the patient is experiencing SVT, inappropriate pacing/shock therapy to treat VT is ineffective, may cause patient discomfort or pain (inappropriate shock), and may actually induce VT. While the patient is experiencing VT, inappropriate pacing/shock therapy to treat SVT may be subject the patient to lengthened time without appropriate VT therapy. Moreover, the efficacy of current pacing therapy to treat SVT is very limited.

In the case that the ventricular rate is high (for example, higher than 150 bpm, the precise value may be defined in advance, in an embodiment patient-dependent) and higher than the atrial rate, a VT is diagnosed without using the invention and appropriate ventricular therapy can be initiated. This possibility is explained for the sake of completeness.

In the case that the ventricular rate is high (i.e. for example higher than 150 bpm, the precise value may be defined in advance, preferably patient-dependent) and the atrial rate is higher than or equal to the ventricular rate during the first time period, a pathological initial ventricular and atrial rate of the heart is detected by the cardiac device. In this case it is possible that the patient suffers a SVT rather than a VT, but VT cannot be excluded as diagnosis yet. To determine if the underlying arrhythmia is SVT or VT, the device triggers a transient release of a diagnostic dose of the antiarrhythmic cardioactive drug in the second period of time, for example, administration of a bolus of adenosine of 6 mg for 1 to 2 seconds.

In this embodiment, immediately after administration of at least one antiarrhythmic cardioactive drug, for example adenosine, the response of the ventricular and atrial rate of the heart within a third time period is detected by the detection unit of the cardiac device.

In case that both, the atrial and ventricular rate response, are detected to be slowed (i.e. for example less than 150 bpm, this value may be user-programmable) within third time period by the detection unit of the cardiac device, an AV nodal dependent SVT is determined, such as AV nodal reentry or bypass-tract-mediated macro reentry, wherein the cardiac device, in particular the control unit, is adapted to determine this arrhythmia type. In this case often the episode is terminated successfully already by the short adenosine pulse and the event is for example logged into a memory of the cardiac device. Additionally, a stability criteria within third time period, for example known from US 2010/0100143 A1 which is included herein by reference, may be used by the detection unit of the cardiac device in order to determine an AV nodal dependent SVT.

In case that only the ventricular rate response has changed (i.e. slowed, wherein slowed means for example less than 150 bpm, this value may be user-programmable) whereas the atrial rate response has not changed compared with the initial ventricular rate and initial atrial rate, respectively, by the detection unit within third time period, an SVT is determined by the control unit of the cardiac device that is not dependent on the AV node such as atrial tachycardia, atrial fibrillation, or atrial flutter.

In case that it is detected by the detection unit that neither the ventricular rate nor the atrial rate has changed within third time period, a VT is determined by the control unit of the cardiac device.

In each case it is then initiated the appropriate therapy to the patient according to the determined type of arrhythmia by the control unit of the cardiac device and/or the cardiac device displays the information with regard to the determined arrhythmia type and/or sends the information to an external unit. Further the determined arrhythmia type may be stored within a memory of the cardiac device for later processing.

In an embodiment the third time period begins immediately after the start of the second time period, preferably 1 to 3 seconds after the start of the second time period or directly after the end of the second time period. This real-time response is for example possible with adenosine. It is also required for quick diagnosis and action.

These as well as other advantages of various aspects of the present invention will become apparent to those of ordinary skill in the art by reading the following detailed description, with appropriate reference to the drawings.

Exemplary embodiments are described herein with reference to the schematic drawings in which:
Fig. 1 illustrates an exemplary embodiment of a device according to the invention; and
Fig. 2 shows a simplified flow chart of an exemplary embodiment of an inventive method.

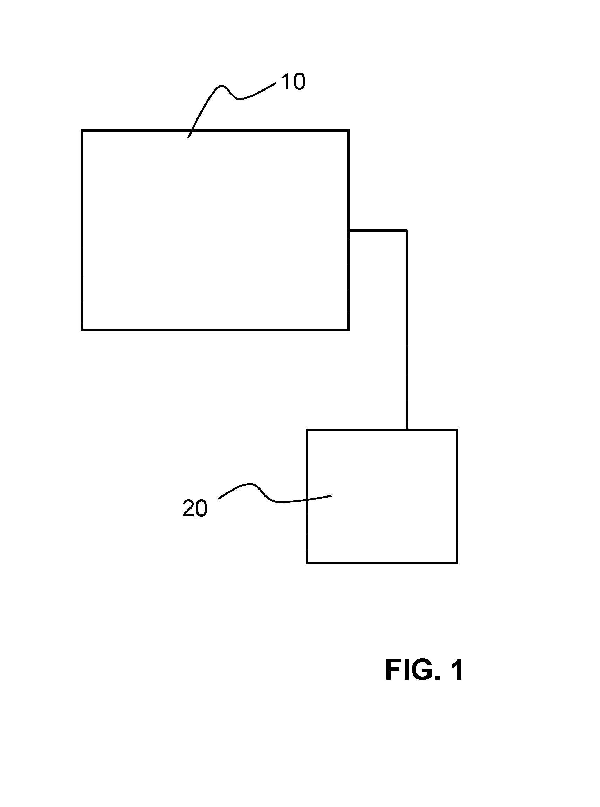

Fig. 1 shows an embodiment of a device for determination of an atrial and ventricular arrhythmia in a patient's heart comprising an implantable cardiac device 10 like a pacemaker, defibrillator or cardioverter, which are in principle known in the art, and a drug delivery device 20 with a pump and a drug depot / reservoir, wherein the drug delivery device 20 may be an implantable or external device. For example, the implantable cardiac device 10 and the drug delivery device 20 may form a combined system as it is described in EP 2 123 326 A1 and US 2009/0292330 A1 which documents are included herein by reference. As the connecting line between the cardiac device 10 and a drug delivery device 20 shows, both devices may communicate wire-connected or not wire-connected with each other via appropriate interfaces.

The cardiac device 10 comprises a power supply, a control unit, a memory and a detection unit which are connected with each other and are not shown in Fig. 1. The detection unit is adapted to sense the atrial and the ventricular rate of the heart of the patient. The control unit processes the signals sensed by the detection unit and is adapted to control the additional functions of the cardiac device, for example the operation of a pacing or shock electrode, and the operation of the drug delivery device 20.

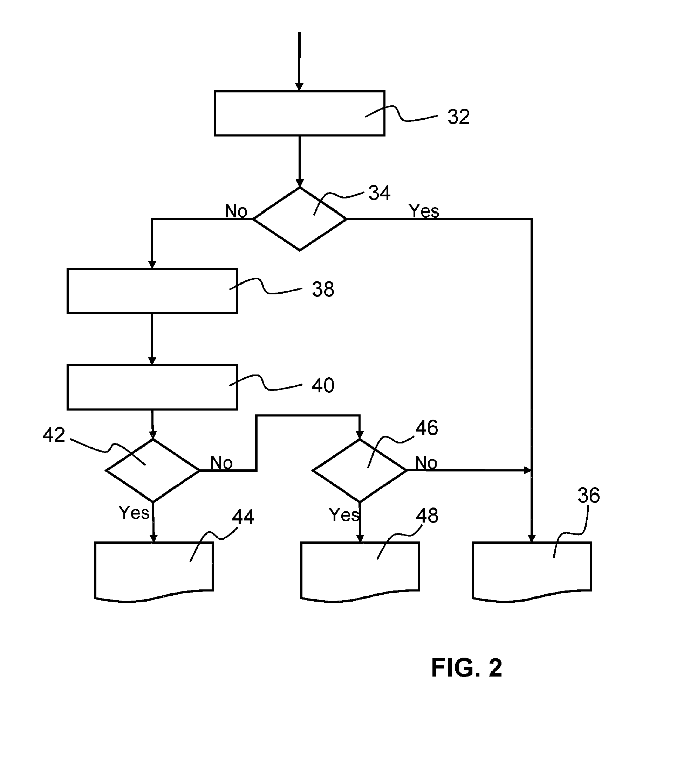

The functionality of the cardiac device 10 and the drug delivery device 20 is explained below with the help of the flowchart shown in Fig. 2.

An embodiment of an inventive method is depicted in Fig. 2. Based on examining the atrial and ventricular response after intravenous release of adenosine a fast diagnostic decision of SVT or VT can be made with high sensitivity and specificity using the method shown in Fig. 2. This method may be carried out utilizing the inventive device shown in Fig. 1.

At first, the detection unit of the cardiac device 10 detects a high ventricular rate in step 32. As explained above, for example 150 bpm (bpm = beats per minute) are regarded as high ventricular rate. Then, the control unit of the cardiac device 10 compares the ventricular rate with the atrial rate in step 34. If the ventricular rate is higher than the atrial rate the procedure continues with the YES branch of the flow diagram and a VT is diagnosed in step 36 by the control unit which then initiates an appropriate ventricular therapy. This therapy may comprise ventricular anti-tachycardia pacing, low energy cardioversion, high energy shock, depended on the programmed tiered therapy settings. Alternatively, a trigger can be generated at the control unit to cause interventions release of certain antiarrhythmic drugs, such as lidocaine, from the drug delivery device 20.

If the atrial rate is higher than or equal to the ventricular rate the procedure continues with step 38 and a decision needs to be made to distinguish SVT from VT arrhythmia. According to the invention, then, the control unit of the cardiac device 10 triggers the pump of the drug delivery device 20 to administer an intravenous dose of adenosine within a second time period. In an embodiment, a dose of 6 mg adenosine is released for one or two seconds.

Directly after administration of the adenosine bolus, i.e. for example 1, 2 or 3 seconds after the begin of the second time period or directly after the end of the second time period, the cardiac device 10 continues monitoring the atrial and ventricular rate over a third period of time (for example 3 to 4 seconds) or over a number of cardiac cycles (e.g. 8 to 10 cycles) in step 40 which are user programmable.

In step 42 the control unit of the cardiac unit 10 decides whether after the adenosine release both the atrial rate and the ventricular rate are slowed within third time period. Therein it is for example determined whether the detected atrial rate and the detected ventricular rate each are below 150 bpm.

If both, the atrial rate and the ventricular rate are slowed the procedure continues with step 44. Therein, a diagnosis is made that the episode is an AV nodal depended SVT, such as AV nodal reentry or bypass-tract-mediated marcoreentry. In most cases, by the short pulse of adenosine, additionally, the episode is terminated successfully and the event is, for example, logged into the memory of the cardiac device 10.

If one of the atrial rate and the ventricular rate is not slowed within third time period, the procedure continues with step 46. In this step the control unit decides whether only the ventricular rate is slowed within third time period. Therein, slowing of the ventricular rate is again decided if the ventricular rate drops below 150 bpm. If the slowing of the ventricular rate occurred, the procedure continues with step 48 wherein it is determined that the diagnosis is SVT which is not dependent on the AV node, such as atrial tachycardia, atrial fibrillation or atrial flutter. Correspondingly, in step 48 an atrial therapy is initiated in order to terminate the SVT. Such therapy may be atrial anti-tachycardia pacing or atrial cardioversion, as known in the art. Alternatively, a trigger may be generated by the control unit to cause intravenous release of certain antiarrhythmic drugs, such as verapamil, diltiazem procainamide, etc. by the pump of the drug delivery device 20, in order to terminate the SVT or to slow the ventricular response.

If neither the atrial rate nor the ventricular rate or rhythm has changed after application of the adenosine bolus (see "NO"-branch beginning at step 46) within third time period then the control unit makes the diagnosis that the episode is a VT arrhythmia (see step 36). An appropriate therapy as explained above is initiated to terminate the VT in step 36.

### Reference Numbers

- 10: implantable cardiac device
- 20: drug delivery device
- 32, 38, 40, 44, 48, 36: steps of inventive method
- 34, 42, 46: steps of inventive method wherein a decision is made

## Claims

1. A method of determination of a type of an atrial and ventricular arrhythmia in a patient's heart, the method comprising:
• monitoring the atrial and ventricular rate of the heart;
• detecting a pathological initial ventricular and/or atrial rate during a first time period;
• administering, preferably intravenous administering, of at least one antiarrhythmic cardioactive drug only over a second time period, preferably over a short time period of 1 to 10 seconds, in case that a pathological initial ventricular and/or atrial rate is detected;
• detecting the response of the ventricular and atrial rate of the heart to the administering of the at least one drug, comparing the ventricular and atrial rate response with the initial ventricular and atrial rate, respectively, within a third time period and determining whether there are changes within the ventricular and/or atrial rate response and determining the type of changes; and
• determining the type of atrial or ventricular arrhythmia from the presence or absence of changes of the atrial and/or ventricular rate response compared with the initial atrial and ventricular rate and from the type of these changes.

2. The method of claim 1 wherein the second time period of administering of the at least one antiarrhythmic cardioactive drug takes 1 to 3 seconds.

3. The method of claim 1 wherein the at least one antiarrhythmic cardioactive drug comprises adenosine.

4. The method of claim 1 wherein in the case that the ventricular rate is high and the atrial rate is higher than or equal the ventricular rate during the first time period a pathological initial ventricular and atrial rate of the heart is detected.

5. The method of claim 4 wherein in the case that both, the atrial and ventricular rate response, are slowed an AV nodal dependent SVT is determined.

6. The method of claim 4 wherein in the case that only the ventricular rate has slowed and the atrial rate response has not changed compared with the initial ventricular rate and initial atrial rate, respectively, an SVT is determined which is not dependent on the AV node.

7. The method of claim 1 wherein the third time period begins shortly after the start of the second time period, preferably 1 to 3 seconds after the start of the second time period.

8. A device for determination of a type of an atrial and ventricular arrhythmia in a patient's heart comprising an implantable cardiac device (10) and a drug delivery device (20), wherein:
• the cardiac device is adapted to monitor the atrial and ventricular rate of the heart;
• the cardiac device is adapted to detect a pathological initial ventricular and/or atrial rate during a first time period;
• the drug delivery device is adapted to administer, preferably intravenous, of at least one antiarrhythmic cardioactive drug only over a short second time period, preferably over a time period of 1 to 10 seconds, in case that a pathological initial ventricular and/or atrial rate is detected;
• the cardiac device is adapted to detect the response of the ventricular and atrial rate of the heart to the administering of the at least one drug, to compare the ventricular and atrial rate response with the initial ventricular and atrial rate, respectively, within a third time period and to determine whether there are changes within the ventricular and/or atrial rate response and to determine the type of changes; and
• the cardiac device is adapted to determine the type of atrial or ventricular arrhythmia from the presence or absence of changes of the atrial and/or ventricular rate response compared with the initial atrial and ventricular rate and from the type of these changes.

9. The device of claim 8 wherein the drug delivering device is adapted to administer the at least one antiarrhythmic cardioactive drug during the second time period over 1 to 3 seconds.

10. The device of claim 8 wherein the at least one antiarrhythmic cardioactive drug comprises adenosine.

11. The device of claim 8 wherein in the case that the ventricular rate is high and the atrial rate is higher than or equal the ventricular rate during the first time period the cardiac device is adapted to detect a pathological initial ventricular and atrial rate of the heart.

12. The device of claim 11 wherein in the case that both, the atrial and ventricular rate response, are slowed the cardiac device is adapted to determine an AV nodal dependent SVT.

13. The device of claim 11 wherein in the case that only the ventricular rate has slowed and the atrial rate response has not changed compared with the initial ventricular rate and initial atrial rate, respectively, the cardiac device is adapted to determine an SVT which is not dependent on the AV node.

14. The device of claim 8 wherein the cardiac device is adapted to the situation that the third time period begins shortly after the start of the second time period, preferably 1 to 3 seconds after the start of the second time period,.
